# EUROPEAN PATENT APPLICATION

(11) **EP 1 493 438 A1**
(43) Date of publication of application: **05.01.2005**
(21) Application number: 03015052.8
(22) Date of filing: 03.07.2003
(51) Int. Cl.: A61K 31/17, A61K 31/55, A61K 31/4178, A61K 31/352, A61P 25/28

(54) **Vanilloid receptor (VR) inhibitors for treatment of Human Immunodeficiency Virus (HIV)-mediated pain states**

(71) Applicant: Bayer HealthCare AG, 51368 Leverkusen (DE)
(72) Inventor: Bouchon, Axel Dr., 42115 Wuppertal (DE); Misawa, Keiko, Nara, 630-8352 (JP)

(57) **Abstract**

The invention relates to the application of Vanilloid receptor (VR) 1 inhibitors for drug development and for the treatment of HIV-mediated neuropathies and neuropathic pain states. Further, the inventor identified a novel signaling cascade connecting the HIV receptor CXCR4 to VR1. Thus, the invention provides molecular evidence that HIV-mediated pain states - initiated upon binding of the virus to CXCR4 - can be inhibited by VR1 antagonists blocking the final execution of the CXCR4NR1 pathway. In addition, the invention demonstrates that present standard therapies for HIV-mediated pain (which do not include VR1 inhibitors) can not interfere with the CXCR4/VR1 pathway thus explaining inefficient patient treatment in the clinics.

## Description

### 1. BACKGROUND OF THE INVENTION

The Acquired Immune Deficiency Syndrome (AIDS) is characterized by susceptibility to infection with opportunistic pathogens, the occurrence of an aggressive form of Human Herpes Virus (HHV)-8-mediated Kaposi's sarcoma or B cell lymphoma, accompanied by a profound decrease in the number of CD4⁺ T cells [Janeway and Travers (1997)]. The Human Immunodeficiency Virus (HIV) is the causative agent of AIDS [Gelmann et al. (1983); Gallo et al. (1983); Barre-Sinoussi (1983)]. HIV is an enveloped retrovirus. Each virus particle has two copies of an RNA genome, which is transcribed into DNA upon infection of target cells and subsequently integrated in the host genome. The RNA transcripts serve both as mRNA to direct the synthesis of novel viruses and later as the genome of newly built virus particles. HIV enters target cells by high-affinity binding of viral envelope protein gp120 to the cellular surface protein CD4. Fusion and entry of the virus depends on the presence of G-protein coupled receptors (GPCRs), such as CCR5 or CXCR4, that acts as cofactors. Physiologically, CCR5 and CXCR4 are stimulated by interaction with the chemokines RANTES, MIP-1β and SDF-1α, respectively [Bleul et al. (1996); Oberlin et al. (1996); Doranz (1996); Deng et al. (1996); Dragic et al. (1996)]. CCR5 and CXCR4 are Gᵢ-Protein coupled receptors that upon stimulation induce various signal transduction pathways [Stantchev and Broder (2001)], in particular mobilization of intracellular Ca²⁺ stores, modulation of Adenylyl Cyclase (AC) activity, induction of Mitogen-activated protein kinases (MAPK) and Phospholipase A 2 (PLA₂) [Audubert et al. (1999); Zhu et al. (1999)]. CCR5 is particularly expressed by macrophages and primary T cells, whereas CXCR4 is primarily expressed on mature CD4⁺ T helper (T_{H}) cells. Early during infection, CD4⁺/CCR5⁺ macrophages are the central target for HIV, whereas later in infection, the viral phenotype switches to a CD4⁺ T_{H}-trophic type. This transformation is followed by a rapid decline of CD4⁺ T cell counts and progression of AIDS [Janeway and Travers (1997)].

In addition to cancer and opportunistic diseases, AIDS patients display a variety of neurological symptoms. These include allodynia, primary and secondary hyperalgesia and different types of sensory and motor neuropathies [Brinley et al. (2001); Brannagan et al.(1997); Hewitt et al. (1997); Griffin et al. (1998); Bouhassira et al. (1999)]. The peripheral neuropathies associated with HIV-1 infection are a diverse group including acute and chronic inflammatory demyelinating polyneuropathy, mononeuropathy multiplex, diffuse infiltrative lymphocytosis syndrome with neuropathy, progressive polyneurophathy, CMV- and HZV-related neuropathies and in particular distal sensory painful polyneuropathy (DSP). DSP becomes symptomatic in very early and later stages of HIV infection and closely correlates to decreased CD4⁺ T cell numbers, increased HIV-load and gp120 concentration in the plasma and the nervous system (NS). On cellular level, DSP is characterized by (i) chronic stimulation and activation of peripheral nociceptive neurons; (ii) neuronal cell death due to pathologically prolonged Ca²⁺ influx from the extracellular space; and (iii) infiltration of activated, inflammatory cells to the site of neuronal destruction [Pardo et al. (2001); Kaul et al. (2001)]. The virus does not infect and replicate in peripheral nerve fibers and DRG neurons which is consistent with the absence of CD4 [Kaul et al. (2001)]. Thus, HIV-mediated nociceptive and neurotoxic effects do not require neuronal infection. However several data suggest a direct neuronal activation by HIV or gp120 [Kaul et al. (2001); Brinley et al. (2001)]. Recently, neuronal CCR5 and CXCR4 expression has been demonstrated, particularly on primary nociceptive neurons and dorsal root ganglion (DRG) neurons [Miller and Meucci (1999)]. Although DRG neurons do not express CD4, it has been previously shown, that gp120 can bind to CXCR4 in a CD4-independent manner [Hesselgesser et al. (1997)]. Upon binding, gp120 excites DRG neurons leading to Ca²⁺ influx, depolarization and the release of nociceptive peptides, such as Substance P [Oh et al. (2001)] and CGRP. Chronic neuronal stimulation by HIV induces Ca²⁺- mediated neuronal apoptosis, necrosis or parapoptosis [Kaul et al. (2001); Leist and Jäättelä (2001); Hesselgesser et al. (1997)]. Thus, it has been suggested that gp120-mediated acute neuronal activation and gp120-associated neurotoxic effects utilizes identical signal transduction pathways [Kaul et al. (2001); Leist and Jäättelä (2001)]. *In vivo,* peripheral [Eron et al. (1996); Oh et al. (2001)] or central [Milligan et al. (2000)] administration of gp120 induces tactile allodynia and thermal hyperalgesia suggesting that CXCR4 is indeed involved in pain perception. In addition, Herzberg and Sagen demonstrated that epineural exposure to gp120 induces neuropathic pain and damage to the gp120-exposed sciatic nerve [Herzberg and Sagen (2001)]. However, a molecular link between HIV-mediated neuronal stimulation, neurotoxicity, neuropathy and neuropathic pain as not been described so far.

HIV-mediated neuropathic pain is currently treated according to the WHO analgetic ladder for treatment of cancer pain. Standard analgetic drugs include Capsaicin, non-steroidal anti-inflammatory drugs (NSAIDs), tricyclic antidepressants, anticonvulsants, mild and strong opoids [Brinley et al. (2001); Glare (2001); Verma (2001); Paice et al. (2000)]. However, although targeting different peripheral and central pain pathways, the therapeutic effects in the clinics have been dissatisfying with below 15% reduction in HIV-mediated neuropathic pain [Brinley et al. (2001)]. Few clinical trails have been conducted so far to prove the efficiency of the currently applied drugs in HIV-mediated neuropathic pain. Remarkably, Amitriptyline and Mexiletine, two drugs frequently used for efficient treatment of cancer pain and diabetic neuropathies, are completely ineffective in clinical trails investigating HIV-mediated neuropathic pain [Shlay et al. (1998); Kieburtz et al. (1998)]. In addition, it is anecdotally reported that Opoids and in particular NSAIDs are less effective in HIV-mediated neuropathic pain compared to other types of neuropathic pain, such as cancer-mediated and diabetes-associated neuropathic pain [Glare (2001); Verma (2001)]. A clinical trail investigating the analgesic effect of NSAIDs and Opoids in HIV-mediated neuropathic pain has not been conducted yet. Further, topically applied Capsaicin has been found to be highly effective in relieving pain associated with various neuropathic pain syndromes, and was mentioned as a possible topical adjuvant analgesic for the relief of DSP in HIV patients [Paice et al. (2000)]. Two possible mechanisms for Capsaicin-mediated pain release are proposed. Firstly, Capsaicin induces acute nociception by stimulating Vanilloid Receptor (VR) 1 [Caterina et al. (1997); Tominaga et al. (1998)] and subsequently desensitizing and downregulating VR1 *in vitro* [Bhave et al. (2002)]. VR1 downmodulation reduces the algesic effects of endogenous VR1 stimuli leading to pain relief. However, in the presence of VR1 phosphorylation ― an event frequently occurring during chronic pain ― Capsaicin-mediated downmodulation is blocked. Under these conditions Capsaicin may act synergistically with endogenous VR1 stimuli resulting in pain amplification [Bhave et al. (2002)]. Secondly, Capsaicin inhibits NF-κB activation in inflammatory immune cells in a VR1-independent fashion and is a potent anti-inflammatory *compound in vivo* [(Sancho et al. (2002); Kim et al. (2003)]. Surprisingly, a randomized, double-masked clinical trail demonstrated, that Capsaicin-treatment of HIV patients with chronic DSP leads to strongly increased pain rather than pain relief [Paice et al. (2000)]. Thus, VR1 may be involved or even directly stimulated during HIV-mediated neuropathic pain. In conclusion, these reports show that standard drugs do not specifically target HIV-activated pain pathways and in addition delineate the high medical need for an efficient treatment of HIV-mediated neuropathic pain.

VR1 (Acc. No. AJ272063 (human), AF029310 (rat)) is a non-specific cation channel with preference for Ca²⁺ expressed preferentially in small sensory neurons and is activated by heat, low pH, phosphorylation and certain ligands. VR1 activation leads to massive Ca²⁺ influx from the extracellular space followed by neuronal depolarization, release of algesic neuropeptides (CGRP and Substance P) and activation of downstream signaling cascades. Chronic stimulation of VR1 induces Ca²⁺-mediated neuronal cell death [Hiura et al. (2002); Jambrina et al. (2003)]. The quaternary structure of VR1 is predominated by homotetramers enabling the cooperative interaction of all stimuli-sensitive and ligand-binding sites [Kedei et al. (2001); Kuzhikandathil et al. (2001); Hui et al. (2003)]. VR1 is considered a molecular sensor that detects various painful stimuli [Caterina et al. (1997); Tominaga et al. (1998)]. Indeed, experiments performed with the VR1 antagonist Capsazepin [Kelly et al. (2002); Walker et al. (2003)] and with VR1-deficient mice [Caterina et al. (2000); Davis et al. (2000)] demonstrated that the receptor is essential for inflammatory pain. Initially, VR1 has been described as receptor for exogenous substances such as Capsaicin and Resiniferatoxin ("Vanilloids") [Caterina et al. (1997)]. In addition, it has been recently demonstrated that metabolic products ("Endovanilloids") of the PLA₂/12-lipoxygenase (LOX)-pathway of arachidonic acid (AA) metabolism can activate VR1. Endovanilloids, such as 12-hydroperoxyeicosatetraenoic acid (12-HPETE), are structurally similar to Capsaicin, and interact with the Capsaicin-binding site [Hwang et al. (2000); Shin et al. (2002)]. Thus, stimuli that induce the production of AA in small sensory neurons lead to the synthesis of endovanilloids responsible for VR1 activation, subsequent neuronal depolarization, neuropeptide release and nociception. The present invention provides the finding, that antagonists of VR1 and of members of the VR1 signaling pathway can be used as pharmaceutical drugs for the treatment of HIV-mediated pain states.

### 2. DETAILED DESCRIPTION OF THE INVENTION

The invention provides mechanistical evidence that gp120/HIV-receptor CXCR4 and VR1 are linked by a novel signal transduction pathway in primary neurons. Upon stimulation with gp120 the pathway subsequently utilizes CXCR4, ERK and p38, PLA2 and 12-LOX leading to the production of endovanilloids. The endovanilloids, 12-HPETE and 12-HETE stimulate VR1 leading to Ca²⁺ influx and release of algesic peptides, such as CGRP. Gp120-mediated CGRP-release can be completely blocked with VR1 antagonists further delineating the role of VR1 for the execution of gp120-induced neuronal stimulation. The pathway is widely independent of other nociceptive signal transduction pathways. Thus, the invention provides (i) a mechanism for HIV-mediated neuropathic pain; (ii) a plausible and likely mechanism for HIV-induced neurotoxicity; (iii) an explanation for the low efficacy of Opoids, anticonvulsants, antidepressants, and NSAIDs, in particular COX inhibitors, in the treatment of HIV-mediated neuropathic pain; and (iv) an explanation for the enhanced pain perception in DSP patients treated with Capsaicin. The invention particularly relates to the use of VR1 inhibitors for drug development and for the treatment of HIV-mediated neuropathy and neuropathic pain. Due to the absence of a satisfying pain therapy, HIV-mediated neuropathic pain is an area displaying the highest medical need.

The invention relates to methods of screening for VR1 antagonists useful for the treatment of HIV-mediated neuropathy or HIV-mediated pain.

A further embodiment of the invention are methods of screening for VR1 antagonists useful for the treatment of HIV-mediated neuropathy or HIV-mediated pain using Ca²⁺-sensitive dyes of bioluminescence assays in cell-free or cellular assay systems.

Furthermore, the invention relates to the use of a VR1 antagonist for the preparation of a pharmaceutical composition for the treatment of HIV-mediated neuropathy or HIV-mediated pain.

The invention also relates to the use of a VR1 antagonist for the preparation of a pharmaceutical composition for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Furthermore, the invention relates to the use of a VR1 antagonist for the preparation of a pharmaceutical composition for the treatment of HIV-mediated neuropathy or HIV-mediated pain, wherein the VR1 antagonist is compound 1.

The invention also relates to the use of compound 1 for the preparation of a pharmaceutical composition for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

A further embodiment of the invention is a pharmaceutical composition containing a VR1 antagonist for the treatment of HIV-mediated neuropathy or HIV-mediated pain.

The invention also relates to a pharmaceutical composition containing a VR1 antagonist for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

The invention also relates to a pharmaceutical composition containing a VR1 antagonist for the treatment of HIV-mediated neuropathy or HIV-mediated pain where the active ingredient is the compound 1.

Furthermore, the invention relates to a pharmaceutical composition containing a VR1 antagonist for the treatment of HIV-mediated neuropathy or HIV-mediated pain where the active ingredient is the compound 1, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention is the use of a VR1 antagonist for the regulation of VR1 activity in a subject having HIV-mediated neuropathy or HIV-mediated pain.

Furthermore, the invention relates to the use of a VR1 antagonist for the regulation of VR1 activity in a subject having HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention are methods of screening for 12-LOX antagonists useful for the treatment of HIV-mediated neuropathy or HIV-mediated pain.

Furthermore, the invention relates to the use of a 12-LOX antagonist for the preparation of a pharmaceutical composition for the treatment of HIV-mediated neuropathy or HIV-mediated pain.

The invention also relates to the use of a 12-LOX antagonist for the preparation of a pharmaceutical composition for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

A further embodiment of the invention is a pharmaceutical composition containing a 12-LOX antagonist for the treatment of HIV-mediated neuropathy or HIV-inediated pain.

The invention also relates to a pharmaceutical composition containing a 12-LOX antagonist for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention is the use of a 12-LOX antagonist for the regulation of 12-Lox activity in a subject having HIV-mediated neuropathy or HIV-mediated pain.

Furthermore, the invention relates to the use of a 12-LOX antagonist for the regulation of 12-LOX activity in a subject having HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention are methods of screening for PLA₂ antagonists useful for the treatment of HIV-mediated neuropathy or HIV-mediated pain:

A further embodiment of the invention is the use of a PLA₂ antagonist for the preparation of a pharmaceutical composition for the treatment of HIV-mediated neuropathy or HIV-mediated pain.

Furthermore, the invention relates to the use of a PLA₂ antagonist for the preparation of a pharmaceutical composition for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

A further embodiment of the invention is a pharmaceutical composition containing a PLA₂ antagonist for the treatment of HIV-mediated neuropathy or HIV-mediated pain.

The invention also relates to a pharmaceutical composition containing a PLA₂ antagonist for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention is the use of a PLA₂ antagonist for the regulation of PLA₂ activity in a subject having HIV-mediated neuropathy or HIV-mediated pain.

Furthermore, the invention relates to the use of a PLA₂ antagonist for the regulation of PLA₂ activity in a subject having HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention are methods of screening for MAPK antagonists useful for the treatment of HIV-mediated neuropathy or HIV-mediated pain.

Furthermore, the invention relates to the use of a MAPK antagonist for the preparation of a pharmaceutical composition for the treatment of HIV-mediated neuropathy or HIV-mediated pain.

The invention also relates to the use of a MAPK antagonist for the preparation of a pharmaceutical composition for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

A further embodiment of the invention is a pharmaceutical composition containing a MAPK antagonist for the treatment of HIV-mediated neuropathy or HIV-mediated pain.

The invention also relates to a pharmaceutical composition containing a MAPK antagonist for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention is the use of a MAPK antagonist for the regulation of MAPK activity in a subject having HIV-mediated neuropathy or HIV-mediated pain.

Furthermore, the invention relates to the use of a MAPK antagonist for the regulation of MAPK activity in a subject having HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with, neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention are methods of screening for CXCR4 antagonists useful for the treatment of HIV-mediated neuropathy or HIV-mediated pain.

Furthermore, the invention relates to the use of a CXCR4 antagonist for the preparation of a pharmaceutical composition for the treatment of HIV-mediated neuropathy or HIV-mediated pain.

The invention also relates to the use of a CXCR4 antagonist for the preparation of a pharmaceutical composition for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

A further embodiment of the invention is a pharmaceutical composition containing a CXCR4 antagonist for the treatment of HIV-mediated neuropathy or HIV-mediated pain.

The invention also relates to a pharmaceutical composition containing a CXCR4 antagonist for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention is the use of a CXCR4 antagonist for the regulation of CXCR4 activity in a subject having HIV-mediated neuropathy or HIV-mediated pain.

Furthermore, the invention relates to the use of a CXCR4 antagonist for the regulation of CXCR4 activity in a subject having HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Another embodiment of the invention are methods of screening for a modulator interfering upstream of CXCR4 ligands useful for the treatment of HIV-mediated neuropathy or HIV-mediated pain.

Furthermore, the invention relates to the use of a modulator interfering upstream of CXCR4 ligands for the preparation of a pharmaceutical composition for the treatment of HIV-mediated neuropathy or HIV-mediated pain.

The invention also relates to the use of a modulator interfering upstream of CXCR4 ligands for the preparation of a pharmaceutical composition for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

A further embodiment of the invention is a pharmaceutical composition containing a modulator interfering upstream of CXCR4 ligands for the treatment of HIV-mediated neuropathy or HIV-mediated pain.

The invention also relates to a pharmaceutical composition containing an antagonist interfering upstream of CXCR4 ligands for the treatment of HIV-mediated neuropathy or HIV-mediated pain, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

Antagonists within the meaning of the invention include but are not limited to proteins, nucleic acids, carbohydrates, antibodies, small molecules, or any other molecule which acts as an antagonist or modulator on the proteins disclosed.

### 2.1 Pharmaceutical Compositions

The antagonists or modulators within the meaning the invention can be incorporated into pharmaceutical compositions suitable for administration of a therapeutic dose of the antagonist or modulator to a mammal or human. Such compositions typically comprise the antagonist or modulator and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration: The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs or hormones.

A pharmaceutical composition of the invention is formulated to be compatible with its intended route of administration. Examples of routes of administration include parenteral, e.g., intravenous, intradermal, subcutaneous, oral, transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin. Sterile injectable solutions can be prepared by incorporating the active compound (e.g., a polypeptide or antibody) in the required amount in an appropriate solvent with one or a combination of ingredients enumerated above, as required, followed by filtered sterilisation. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatine capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, arid polylactic acid. Methods for the preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration. For pharmaceutical compositions which include an antagonist of VR1, a compound which reduces VR1 expression, or a compound which reduces expression or activity of a protein in the VR1 signaling pathway or any combination thereof, the instructions for administration will specify use of the composition for HIV-mediated pain states.

### 2.2. Determination of a Therapeutically Effective Dose

The determination of a therapeutically effective dose is well within the capability of those skilled in the art. For any compound, the therapeutically effective dose can be estimated initially either in cell culture assays or in animal models, usually rats, rabbits, dogs, or pigs. The animal model also can be used to determine the appropriate concentration range and route of administration. Such information can then be used to determine useful doses and routes for administration in humans.

Therapeutic efficacy and toxicity, e.g., ED₅₀ (the dose therapeutically effective in 50% of the population) and LD₅₀ (the dose lethal to 50% of the population), can be determined by standard pharmaceutical procedures in cell cultures or experimental animals. The dose ratio of toxic to therapeutic effects is the therapeutic index, and it can be expressed as the ratio, LD₅₀/ED₅₀. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED₅₀ with little or no toxicity. The dosage varies within this range depending upon the dosage form employed, sensitivity of the patient, and the route of administration. The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered once or twice daily every 3 to 4 days, every week, or once every two weeks depending on the half-life and clearance rate of the particular formulation.

Normal dosage amounts can vary from 0.1 micrograms to 100,000 micrograms, up to a total dose of about 1 g, depending upon the route of administration. Guidance as to particular dosages and methods of delivery is provided in the literature and generally available to practitioners in the art.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.^

### 3. DESCRIPTION OF THE FIGURES

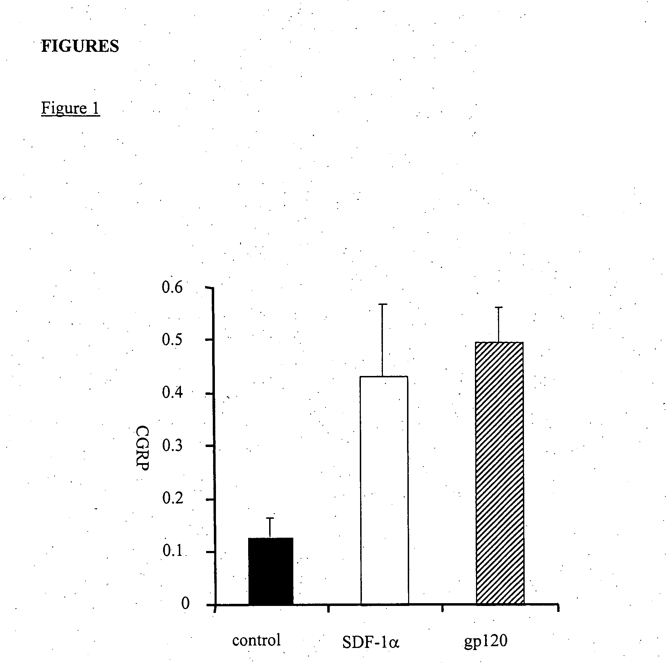

### Figure 1: CGRP-release from primary rat DRG neurons upon stimulation with gp120, SDF-1α or control peptide.

DRG neurons were isolated from adult male Wistar rats and incubated at 37°C, 5% CO₂ for 36 h at a concentration of 8000 DRG neurons/stimulation point. After washing once with RAB buffer, cells were stimulated for 10 min with control peptide (250 pg/ml), gp120 HIV-1_{IIIB} (250 pg/ml) or SDF-1α (1 ng/ml) as indicated. Supernatants were removed and analyzed by EIA for CGRP release.

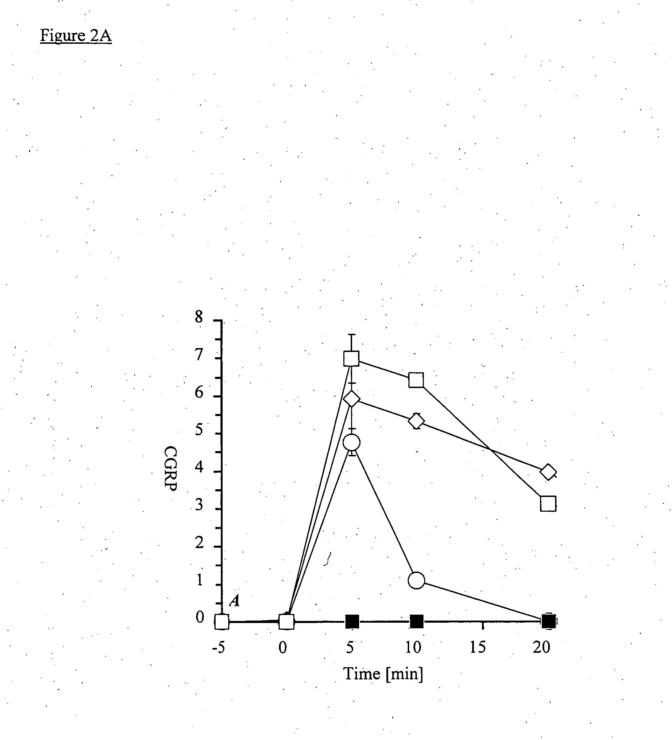

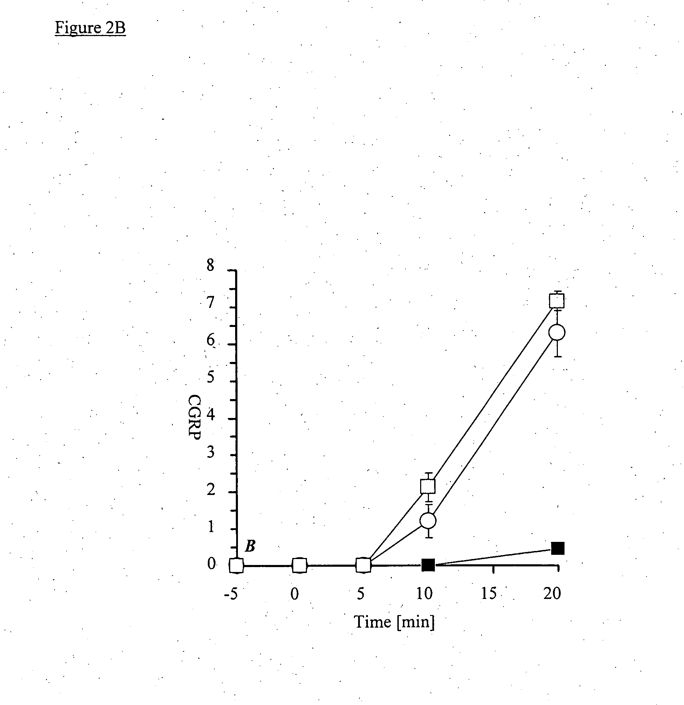

### Figure 2: Kinetics of CGRP-release from isolated rat sciatic nerve upon VR1 stimulation or stimulation with gp120 or SDF-1α.

Sciatic nerve was isolated from adult male Wistar rats, fixed on a polystyrol rod and maintained in RAB buffer until 5 min before stimulation (time point ― 5).
(A) RAB buffer was exchanged, the nerve incubated for 5 min and subsequently stimulated (time point 0) with Capsaicin (100 nM in RAB buffer; white diamonds), low pH (RAB buffer pH 4.5; white squares), RAB buffer heated to 55°C (white circles) or RAB buffer pH 7.4 as control (black squares).
(B) RAB buffer was exchanged, the nerve incubated for 5 min and subsequently stimulated (time point 0) with control peptide (250 pg/ml; black squares), SDF-1α (1 ng/ml; white squares) or gp120 HIV-1_{IIIB} (250 pg/ml; grey squares) in RAB buffer pH 7.4.

After 5 min, 10 min and 20 min supernatant was substituted with fresh RAB buffer pH 7.4 (timepoint 5 and 10, respectively) and the samples placed on ice until analysis. All samples were analyzed by EIA for CGRP release and normalized by the dry weight of the particular sciatic nerve.

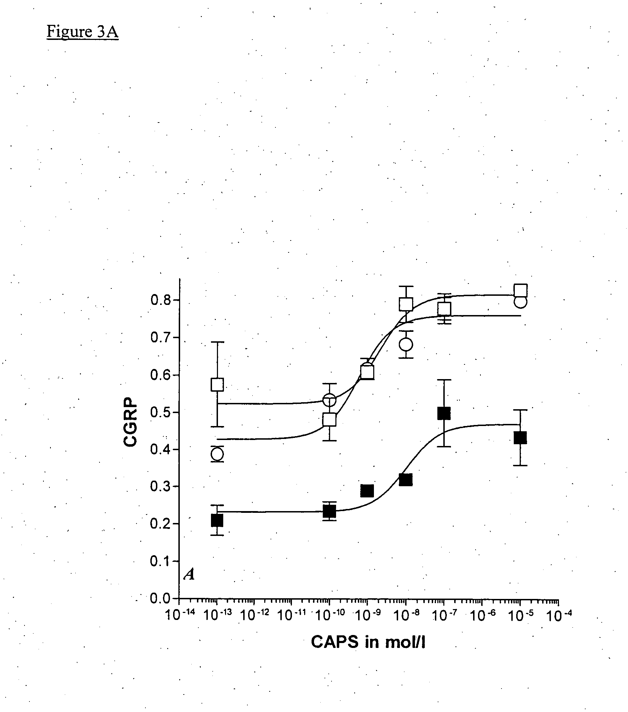

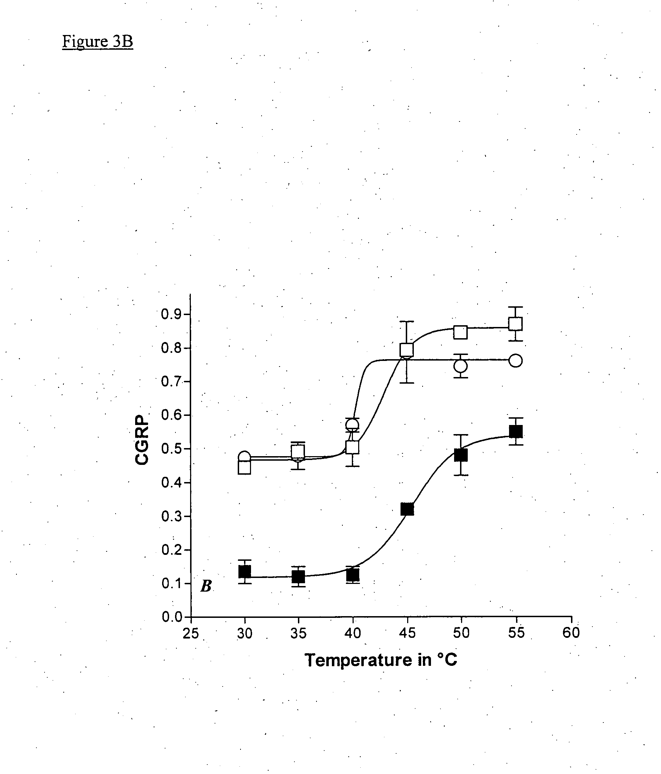

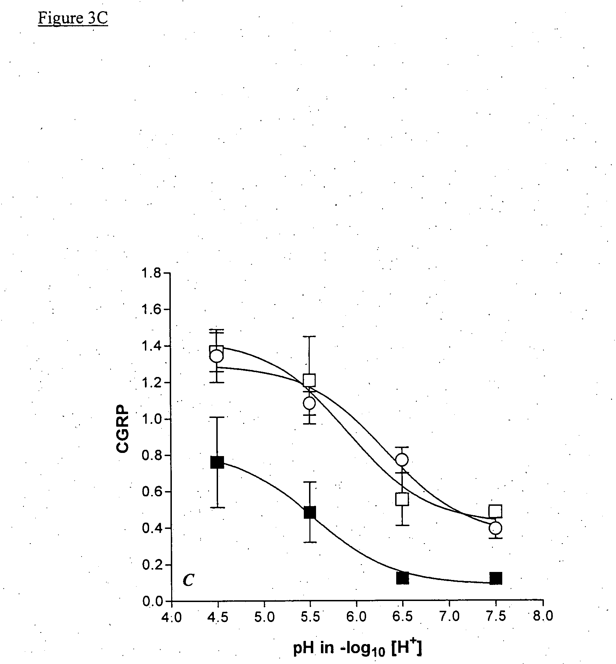

### Figure 3: Sensitization of Capsaicin-, low pH- or temperature-induced VR1 activation from rat DRG neurons by gp120.

DRG neurons were isolated from adult male Wistar rats and incubated at 37°C, 5% CO₂ for 36 h at a concentration of 8000 DRG neurons/stimulation point. After washing once with RAB buffer, cells were stimulated for 10 min with control peptide (250 pg/ml; black squares), gp120 HIV-1_{IIIB} (250 pg/ml; white squares) or SDF-1α (1 ng/ml; white circles) in the presence of different Capsaicin concentrations (A), various temperatures (B) and pH values (C). Supernatants were removed and analyzed by EIA for CGRP release.

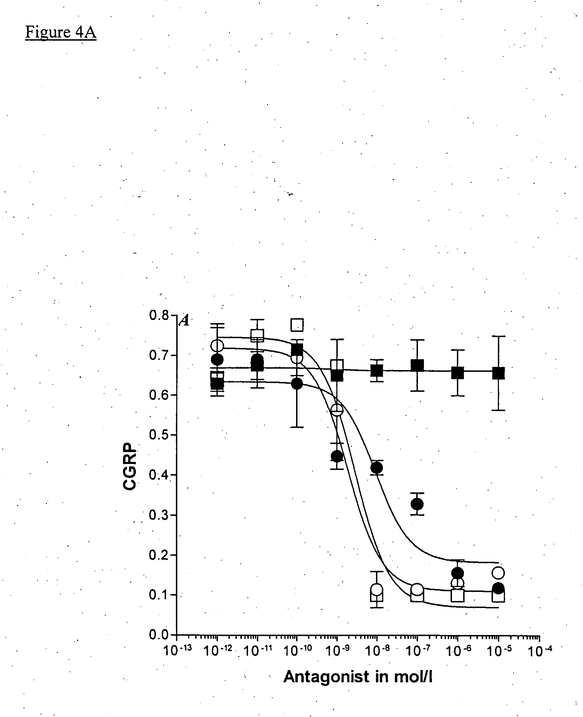

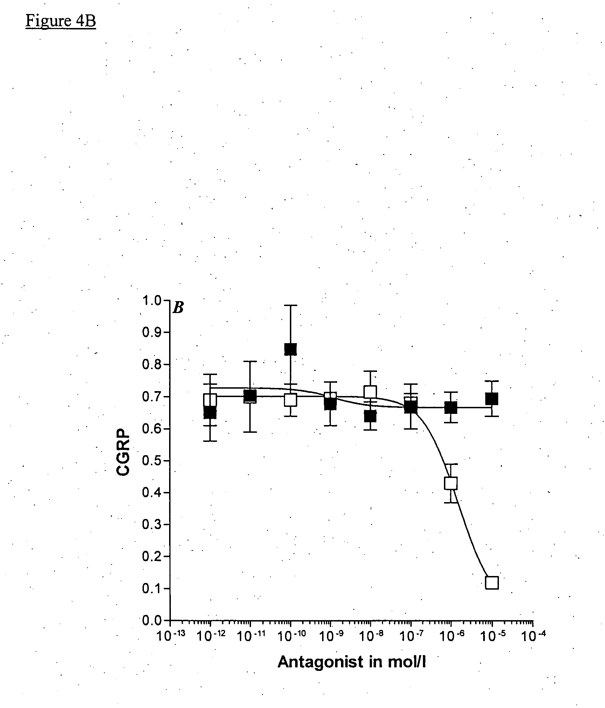

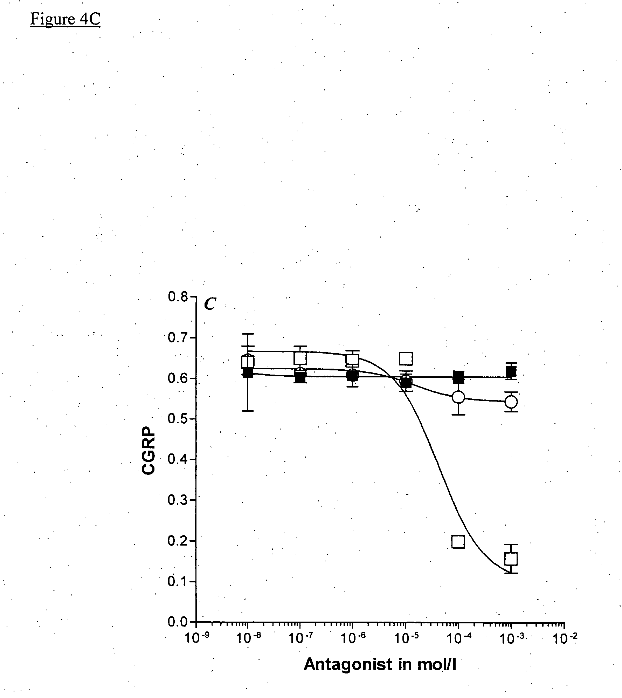

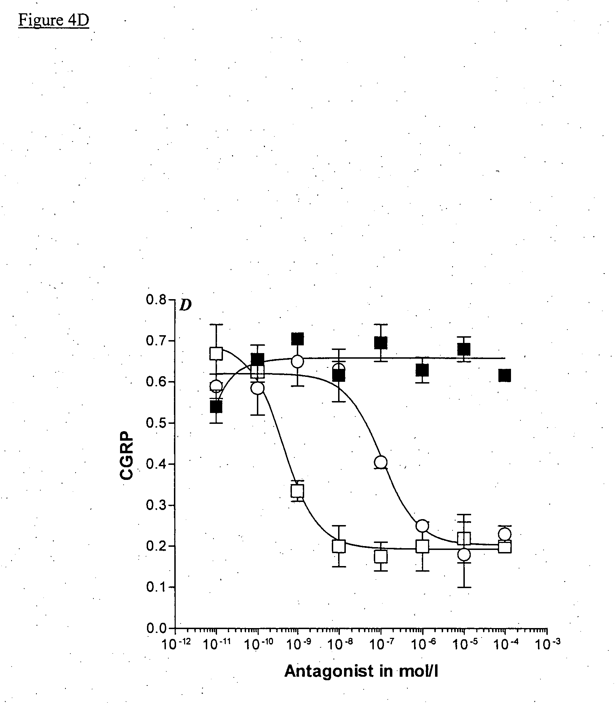

### Figure 4: Inhibition of gp120- mediated CGRP-release from rat DRG neurons by PD98059, U0126, SB203580, QN, NDGA, Ibuprofen, Capsazepin and compound of example 1.

DRG neurons were isolated from adult male Wistar rats and incubated at 37°C, 5% CO₂ for 36 h at a concentration of 8000 DRG neurons/stimulation point. After washing once with RAB buffer, cells were stimulated for 10 min with gp120 HIV-1_{IIIB} alone (250 pg/ml; black squares) or in the presence of the indicated concentrations of (A) SB203580 (p38 Inh.; white squares), PD98059 (MEK-1 Inh.; black circles) and U0126 (MEK-1 Inh.; white circles); (B) Quinacrine (PLA₂ Inh.; white squares); (C) NDGA (LOX Inh.; white squares); Ibuprofen (COX Inh.; white circles); and (D) Capsazepin (VR1 Inh.; white circles) and compound 1 (VR1 Inh.; white squares). Supernatants were removed and analyzed by EIA for CGRP release.

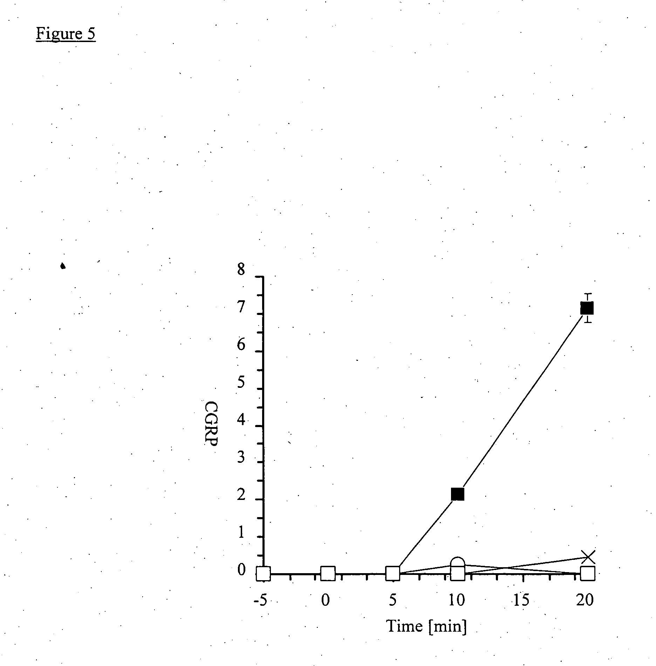

### Figure 5: Inhibition of gp120-mediated CGRP release form rat sciatic nerve by Capsazepin and compound of example 1.

Sciatic nerve was isolated from adult male Wistar rats, fixed on a polystyrol rod and maintained in RAB buffer until 5 min before stimulation (time point ― 5). RAB buffer was exchanged, the nerve incubated for 5 min and subsequently stimulated for 10 min with gp120 HIV-1_{IIIB} (250 pg/ml; black squares) or control peptide (250 pg/ml; black crosses) in the presence of Capsazepin (1 µM; white circles), compound of example 1 (20 nM; white squares) or 0.1% DMSO as control (black squares and crosses). After 5 min, 10 min and 20 min supernatant was substituted with fresh solution or RAB buffer pH 7.4 and the samples placed on ice until analysis. All samples were analyzed by EIA for CGRP release and normalized by the dry weight of the particular sciatic nerve.

### 4. EXAMPLES

The present invention is based on the inventors' identification of a hovel signaling pathway in primary nociceptive sensory neurons connecting CXCR4, the receptor for SDF-1α and the HIV protein gp120, with VR1. Thus, VR1 antagonists can interfere with SDF-1α-, HIV- and gp120-mediated neuronal excitation and inhibit neuropathic pain perception, particularly in HIV and AIDS patients.

### 4.1. Example 1

### 4.1.1. Compound 1

N-(7-hydroxy-5,6,7,8-tetrahydro-naphthalen-1-yl)-N'-(4-trifluoromethoxy-benzyl)-urea 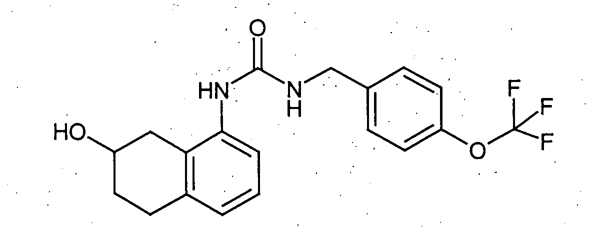 This example was performed according to the general Method A (2.2.1).

A mixture of 7-hydroxy-5,6,7,8-tetrahydro-naphthalen-1-yl)-carbamic acid phenyl ester (30.0 mg, 0.11 mmol) and 4-trifluoromethoxy-benzylamine (21.3 mg, 0.11 mmol) in DMSO (1.0 ml) was stirred at 100°C for 17 hours. The reaction mixture was cooled to room temperature, and water was added. Precipitates were filtered and washed with water then with acetonitrile to obtain N-(7-hydroxy-5,6,7,8-tetrahydro-naphthalen-1-yl)-N'-(4-trifluoromethoxy-benzyl)-urea (6.70 mg, 17 % yield).

¹H NMR (DMSO-*d*₆) δ 1.54-1.65 (m, 1H), 1.81-1.92 (m, 1H), 2.25-2.38 (m, 1H), 2.68-2.88 (m, 3H), 3.86-3.98 (m, 1H), 4.32 (d, *J*= 6.0 Hz, 2H), 4.85 (d, *J* = 4.1 Hz, 1H), 6.72 (d, *J* = 7.5 Hz, 1 H), 6.98 (t, *J* = 7.5 Hz, 1H), 7.06 (t, *J* = 6.0 Hz, 1H), 7.34 (d, *J* = 8.3 Hz, 2H), 7.43 (d, *J* = 8.3 Hz, 2H), 7.63 (d, *J* = 7.5 Hz, 1 H), 7.5 (s, 1H).
Molecular weight: 380.36
MS (M+H): 381
mp: 213°C

### 4.1.2. Method A (Synthesis of Compound 1)

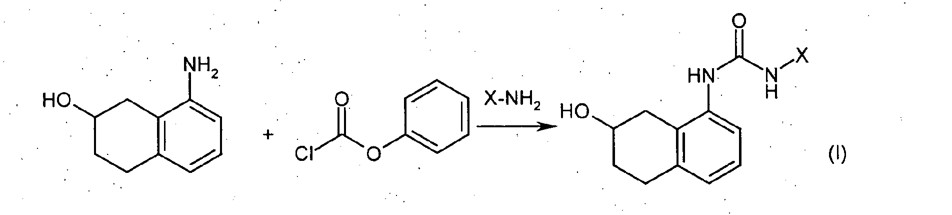

The compound (I) can be prepared by (1) reacting 8-amino-1,2,3,4-tetrahydronaphthalen-2-ol and phenyl chloroformate, and (2) adding amine represented by the formula X-NH₂ to the reaction mixture. The reaction (1) may be carried out in a solvent including, for instance, ethers, such as dioxane, and tetrahydrofuran; aromatic hydrocarbons such as benzene, toluene and xylene; nitriles such as acetonitrile; amides such as dimethylformamide (DMF) and dimethylacetamide; sulfoxides such as dimethyl sulfoxide, and others.

The reaction temperature can be optionally set depending on the compounds to be reacted. The reaction temperature is usually, but not limited to, about 20°C to 50°C. The reaction may be conducted for, usually, 30 minutes to 10 hours and preferably 1 to 24 hours.

The reaction (2) may be carried out in a solvent including, for instance, ethers, such as dioxane, and tetrahydrofuran; aromatic hydrocarbons such as benzene, toluene and xylene; nitriles such as acetonitrile; amides such as dimethylformamide (DMF) and dimethylacetamide; sulfoxides such as dimethyl sulfoxide, and others.

The reaction temperature can be optionally set depending on the compounds to be reacted. The reaction temperature is usually, but not limited to, about 30°C to 120°C. The reaction may be conducted for, usually, 1 hour to 48 hours and preferably 2 to 24 hours.

The 8-amino-1,2,3,4-tetrahydro-naphthalen-2-ol can be prepared by the use of known techniques, and phenyl chloroformate and amine are commercially available or can be prepared by the use of known techniques.

### [Starting compound A]

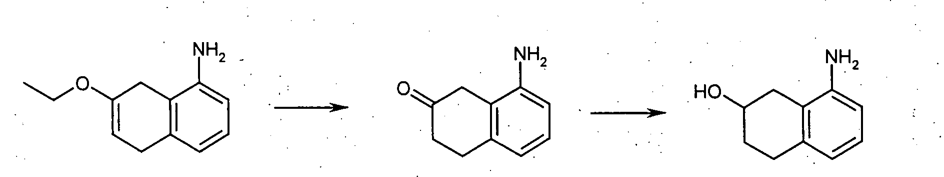

To a stirred solution of 7-ethoxy-5,8-dihydronaphthalen-1-ylamine (1.07 g, 5.65 mmol) in tetrahydrofuran (30 mL) was added solution of aqueous 2N HCl (10 mL), and stirred at 40°C for 1 hour. The mixture was neutralized with addition of sodium bicorbonate, and the product was extracted with ethylacetate. The organic layer was washed with water, dried over Na₂SO₄, filtered, and concentrated under reduced pressure to afford 8-amino-3,4-dihydro-1H-naphthalen-2-one (0.71 g, 78 % yield).
**MS (ESI) m/z 162 [M+H]**^{**+**}
¹H NMR (CDCl₃) δ 2.62-2.65 (m, 2H), 3.07 (t, *J* = 7.25 Hz, 2H), 3.34(s, 2H), 6.65 (d, *J=* 7.85, 1H), 6.70 (d, *J=* 7.25 Hz, 1H), 7.07 (t, *J* = 7.55 Hz, 1H).

Next, to 8-amino-3,4-dihydro-1H-naphthalen-2-one (0.050 g, 0.318 mmol) in methanol (10 mL) was added sodium borohydride (0.030 g, 0.175 mmol) at 0°C, and the mixture was stirred for 1 hour. The mixture was poured into water, and the product was extracted with ethylacetate. The organic layer was dried over Na₂SO₄, filtered, and concentrated under reduced pressure to afford 8-amino-1,2,3,4-tetrahydro-naphthalen-2-ol (0.037 g, 71 % yield).
**MS (ESI) m/z 163 [M]**^{**+**}
¹H NMR (DMSO-*d6*) δ 1.53-1.57 (m, 1H), 1.81-1.85 (m, 1H), 2.16 (dd, *J* = 7.7 and 16.4 Hz, 1H), 2.61-2.74 (m, 3H), 3.89-3.90 (m, 1H), 4.65 (s, 2H), 4.72 (d, *J* = 4.1 Hz, 1H), 6.28 (d, *J =* 7.45 Hz, 1H), 6.28 (d, *J* = 7.45 Hz, 1H), 6.41 (d, *J* = 7.7 Hz, 1H), 6.76(t, *J* = 7.55 Hz, 1H).

### 4.2. Example 2

### 4.2.1. Methods of screening for VR1 antagonists

### 4.2.1.1 Fluorescent Ca²⁺ indicators excited with UV light

Fluorescent probes showing a spectral response upon Ca²⁺ binding enables the detection of changes in the intracellular free Ca²⁺ concentration by fluorescence mircoscopy, flow cytometry and fluorescence spectroscopy. The fluorescent, UV-light excitable indicators are mostly derivatives of the Ca²⁺ chelators EGTA, APTRA and BABTA, such as fura-2, indo-1, fura-4F, fura-5F, fura-6F, fura-FF, fura-red, Calcium Green, Oregon Green 488 BABTA. Such indicators can be used for the screening of VR1 antagonists using cell-free systems or cellular systems containing primary cells or VR1-transfected cell lines [O'Malley et al. (1999)].

### 4.2.1.2 Fluorescent Ca²⁺ indicators excited with visible light

Fluorescent probes, excitable by visible light, such as fluo-3, fluo-4, Rhod-2, X-Rhod-1 showing a spectral response upon Ca²⁺ binding enables the detection of changes in the intracellular free Ca²⁺ concentration by laser-based instrumentation, such as flowcytometers or laser-scanning microscopes. Such indicators can be used for the screening of VR1 antagonists using cell-free systems or cellular systems containing primary cells or VR1-transfected cell lines [O'Malley et al. (1999)].

### 4.2.1.3 Bioluminescent Ca²⁺ indicators

Bioluminescence is defined as the production of light by biological organsims which does not require illumination. Transfection of VR1-expressing cells with the photoprotein aequorin form the jellyfish *Aequorea victoria* can be used for screening of VR1 antagonists. The aequorin complex comprises apoaquorin protein, molecular oxygen and the luminophore coelenterazine. When Ca²⁺ binds to the complex, coelenterazine is oxidized to coelenteramide, with a concomiant release of carbon dioxide and blue light. Unlike fluorescent Ca²⁺ indicators, Ca²⁺-bound aequorin can be detected without illuminating the sample. Thus, agonist-induced, VR-1-mediated Ca²⁺-influx can be detected by increased generation of light, whereas VR1 inhibitors antagonize VR1-mediated Ca²⁺-influx and light production [Miller et al. (1994)].

### 4.3 Example 3

### 4.3.1 gp120-mediated CGRP-release from DRG neurons and N. Ischadicus

The invention provides evidence that SDF-1α and gp120 induce release of CGRP from rat DRG neurons and isolated rat sciatic nerves *ex vivo* (Table 1 and 2; Figure 1 and 2). This is the first evidence that gp120 can directly stimulate primary peripheral nerves and neurons supporting the hypothesis that direct HIV interaction with neurons is responsible for HIV-associated neuropathies and not secondary effects mediated by HIV-infected immune cells [Kaul et al. (2001)].

**Table 1:**

| CGRP-release ([OD₄₀₅]) from rat DRG neurons (2.3.2. Method B) | | |
|---|---|---|
| **control** | **SDF-1α** | **gp120** |
| 0.13+/-0.03 | 0.43+/-0.14 | 0.5+/-0.07 |

### Figure 1: CGRP-release from primary rat DRG neurons upon stimulation with gp120, SDF-1α or control peptide (2.3.2. Method B).

DRG neurons were isolated from adult male Wistar rats and incubated at 37°C, 5% CO₂ for 36 h at a concentration of 8000 DRG neurons/stimulation point. After washing once with RAB buffer, cells were stimulated for 10 min with control peptide (250 pg/ml), gp120 HIV-1_{IIIB} (250 pg/ml) or SDF-1α (1 ng/ml) as indicated. Supernatants were removed and analyzed by EIA for CGRP release.

**Table 2A:**

| CGRP-release ([ng CGRP/mg nerve dry weight]; t₀-t₅: stimulation period) from rat sciatic nerve (2.3.3. Method C) | | | | |
|---|---|---|---|---|
| **Time [min]** | **control** | **CAPS** | **50°C** | **pH 4.5** |
| - 5 | 0 | 0 | 0 | 0 |
| 0 | 0 | 0.05923 ng/mg | 0 | 0 |
| 5 | 0 | 5.9+/-1.3 ng/mg | 4.8+/-0.4 ng/mg | 7.0+/-0.7 ng/mg |
| 10 | 0 | 5.3+/-0.2 ng/mg | 1.1+/-0.2 ng/mg | 6.4+/-0.2 ng/mg |
| 20 | 0 | 4.0+/-0.1 ng/mg | 0 | 3.1+/-0.1 ng/mg |

**Table 2B:**

| CGRP-release ([ng CGRP/mg nerve dry weight]; t₀-t₁₀: stimulation period) from rat sciatic nerve (2.3.3. Method C) | | | |
|---|---|---|---|
| **Time [min]** | **control** | **SDF-1α** | **gp120** |
| -5 | 0 | 0 | 0 |
| 0 | 0 | 0 | 0 |
| 5 | 0 | 0 | 0 |
| 10 | 0 | 1.2+/-0.5 ng/mg | 2.1+/-0.4 ng/mg |
| 20 | 0.4+/-0.1 ng/mg | 6.3+/-0.7 ng/mg | 7.2+/-0.3 ng/mg |

### Figure 2: Kinetics of CGRP-release from isolated rat sciatic nerve upon VR1 stimulation or stimulation with gp120 or SDF-1α (2.3.3. Method C).

Sciatic nerve was isolated from adult male Wistar rats, fixed on a polystyrol rod and maintained in RAB buffer until 5 min before stimulation (time point ― 5).

(C) RAB buffer was exchanged, the nerve incubated for 5 min and subsequently stimulated (time point 0) with Capsaicin (100 nM in RAB buffer; white diamonds), low pH (RAB buffer pH 4.5; white squares), RAB buffer heated to 55°C (white circles) or RAB buffer pH 7.4 as control (black squares).

(D) RAB buffer was exchanged, the nerve incubated for 5 min and subsequently stimulated (time point 0) with control peptide (250 pg/ml; black squares), SDF-1α (1 ng/ml; white squares) or gp120 HIV-1_{IIIB} (250 pg/ml; grey squares) in RAB buffer pH 7.4.

After 5 min, 10 min and 20 min supernatant was substituted with fresh RAB buffer pH 7.4 (timepoint 5 and 10, respectively) and the samples placed on ice until analysis. All samples were analyzed by EIA for CGRP release and normalized by the dry weight of the particular sciatic nerve.

### 4.3.2 Method B: CGRP-release from rat DRG neurons

### 4.3.2.1 Preparation of DRG cell culture plates

96-well cell culture plates for cultivation of DRG neurons are coated with Poly-D-Lysin (GibcoBRL, Grand Island, NY, USA) (50 µg/ml F12 Medium, 50µl/well, 30min at RT, washed 3 x with 200µl sterile water dry completely) and Laminin (GibcoBRL, Grand Island, NY, USA) (5 µg/ml F12 Medium, 30 µl/96-well, 30 min at RT, wash 3 x with 200µl sterile water, dry completely). Upon drying 96well plates can be used directly or be stored at RT for at least 7 days.

### 4.3.2.2 Preparation of rat spinal column and DRGs

Male Wistar rats (180-300 g) are sacrificed by decapitation and bled completely. Upon ventral opening, organs and ribs are removed. In the cranial area the spine is separated from muscle and skin using a scalpel. For caudal separation from the lower lumbar vertebra a pair of bone scissors is used. Further steps of the preparation are performed in ice-cold medium under a clean bench. The spine is divided in three equal pieces and the spine channel is opened first dorsally than ventrally along the medians leading to six spine fragments. Spinal cord and dura mater are removed with tweezers to grant excess to the capsule of DRGs. Under microscopic control the DRGs can be separated from the peripheral nerve and excess of connecting tissue using a pair of spring scissors and tweezers. Separated DRGs are subsequently transferred to a small dish containing ice-cold medium. Under optimal conditions 2x10⁶ DRGs/rat can be isolated:

### 4.3.2.3 Isolation and cultivation of DRG neurons

DRGs are incubated in 4 ml of Collagenase solution for 2 h at 37°C (1.25 mg/ml Collagenase in DMEM (GibcoBRL, Grand Island, NY, USA)), 4 ml Trypsin-EDTA solution for 30 min at 37°C (GibcoBRL, Grand Island, NY, USA)), and DNase solution for 5 min at 37°C (1 mg/ml DNase (GibcoBRL, Grand Island, NY, USA) in DMEM). Cells are finally washed twice in DRG-Medium (Nutrient Mix F12, 10% Horse Serum, 200mM Glucose, 2mM Glutamine (all GibcoBRL, Grand Island, NY, USA)), counted and plated at a concentration of 30000 cells/well. After over night incubation medium containing cell debris and dead cells is substituted.

### 4.3.2.4 DRG release assay

DRG neurons are gently washed once with Release Assay Buffer (RAB: 138 mM NaCl, 5 mM KCl, 1.2 mM MgCl₂, 2 mM CaCl₂; 10 mM Glucose, 10 mM HEPES pH 7.4; all from Sigma, St. Louis, MO, USA) and stimulated with Capsaicin solution (100 nM Capsaicin in RAB pH 7.4 (Sigma)), RAB pH 4.5 or preheated RAB pH 7.4 (50°C) in the presence or absence of inhibitors. After 5 or 10 min of stimulation samples are neutralized with NaOH if necessary (stimulation with RAB pH 4.5; neutralization: 3.7µl NaOH (200 mM) to 110µl RAB pH 4.5) and the supernatant transferred to EIA plates (CGRP Release Kit, SpiBio, Paris, France). EIA is performed according to the manufacturer's protocol (CGRP Release Kit, SpiBio, Paris, France).

### 4.3.3 Method C: CGRP-release from rat Nervus Ischadicus

### 4.3.3.1 Preparation of Nervus ischiadicus (modified after [Sauer et al. (2001)])

Male Wistar rats (180-220 g) are anesthetized using Isofluran, Dinitricoxide and Oxygen (5:68:27 (v/v/v)) in a vaporisator. The hind legs are shaved and opened along the femur. Using tweezers and scissors the *Musculus gluteus superficialis* and *Musculus bicepsfemoris* are separated gaining access to the *Nervus ischiadicus*. The *Nervus ischiadicus* is cut proximally 1 cm before entry into the spine and laterally at the branching of *Nervus fibularis* and *Nervus tibialis*. Dependent on the size and weight of the animal, the length of the preparation is between 2 - 3 cm. The nerve is coiled around a polystyrol spatula (Rührspatel, Sarstedt, Germany) and fixed at the ends with cyanacrylate glue (Loctite 401, Loctite GmbH, Germany). The fixed nerve is transferred to a 1.5 ml eppendorf tube containing 300 µl RAB, pH 7.4 at RT.

### 4.3.3.2 Stimulation of Nervus ischiadicus and CGRP-release assay (modified after [Sauer et al. (2001)])

To reset all samples, sciatic nerve preparations are transferred 5 min prior to stimulation to fresh 300 µl of RAB pH 7.4 (prestimulation ― point zero). Stimulation is performed for 5 or 10 min with Capsaicin solution (100 nM Capsaicin in RAB pH 7.4), RAB pH 4.5 or preheated RAB pH 7.4 (50°C) in the presence or absence of inhibitors. To evaluate post-stimulation CGRP-release sciatic nerve preparations are transferred in 5-min-intervals to fresh tubes containing RAB pH 7.4. Supernatants of each sample are transferred to EIA plates (CGRP Release Kit, SpiBio, Paris, France) and analyzed in triplicate (3 x 100µl) according to the manufacterer's protocol. Upon completion of the experiment the sciatic nerve preparations are carefully removed from the spatula, dried on 3MM paper and weighed. CGRP concentrations obtained from the CGRP-release EIA are finally normalized to the dry weight of the sciatic nerve preparations.

### 4.4 Example 4

The invention provides evidence that gp120 and SDF-1α can sensitize VR1 for stimulation with Capsaicin, low pH and heat (Table 3; Figure 3). Thus gp120-stimulated pathways directly affect and modify the molecular parameters (EC₅₀, EpH₅₀, ET₅₀) of VR1. Further, these data demonstrate that gp120-initiated pathways are linked to VR1 and operate upstream of VR1 activation instead of acting synergistically to VR1-mediated pathways in an parallel manner. The observation that Capsaicin sensitizes for gp120-mediated CGRP release explains the algesic effect of topical Capsaicin application in HIV patients with DSP [Paice et al. (2000)]. Apparently, in this clinical setting VR1 is unable to be desensitized by Capsaicin since neurons are already chronically stimulated downstream of gp120 [Bhave et al. (2002)]. Thus, Capsaicin amplifies gp120-mediated algesic effects instead of acting analgesic.

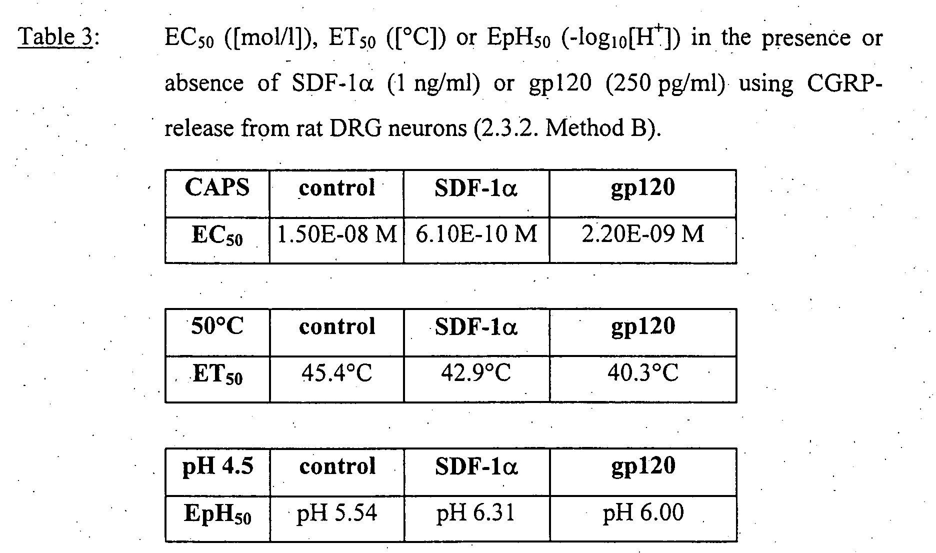

### Figure 3: Sensitization of Capsaicin-, low pH- or temperature-induced VR1 activation from rat DRG neurons by gp120 (2.3.2 Method B).

DRG neurons were isolated from adult male Wistar rats and incubated at 37°C, 5% CO₂ for 36 h at a concentration of 8000 DRG neurons/stimulation point. After washing once with RAB buffer, cells were stimulated for 10 min with control peptide (250 pg/ml; black squares), gp120 HIV-1_{IIIB} (250 pg/ml; white squares) or SDF-1α (1 ng/ml; white circles) in the presence of different Capsaicin concentrations (A), various temperatures (B) and pH values (C). Supernatants were removed and analyzed by EIA for CGRP release.

### 4.5 Example 5

The invention provides data that gp120-mediated CGRP release from DRG neurons can be completely inhibited by the following specific inhibitors: (i) the MEK-1 inhibitors PD98059 and U0126 (Table 4; Figure 4A); (ii) the p38 inhibitor SB203580 (Tabel 4; Figure 4A) (iii) the PLA₂ inhibitor Quinacrine (QN) (Table 4; Figure 4B); (iv) the 12- and 15-LOX inhibitor Nordihydroguaiaretic Acid (NDGA) (Table 4; Figure 4C) and (v) the VR1 inhibitors Capsazepin (CAZ) (Table 4; Figure 4D) and the Cpd 1 (Table 4; Figure 4D). Thus, the invention clarifies the complete molecular connection from CXCR4 to VR1-stimulation and VR-1-mediated release of algesic CGRP identifying several potential targets for the intereference with gp120-mediated neuronal stimulation. Thus, gp120-mediated neuronal excitation is initiated by interaction with CXCR4 and executed by VR1 activation. The data mechanistically explain the observation, that gp120-induced neuropathy in rats can be antagonized with p38 inhibitors [Kaul et al. (2001)]. Particularly, the inhibition of gp120-mediated CGRP release with VR1 antagonists (Capsazepin and Cpd. 1) opens the possibility to treat HIV-associated neuropathic pain and neuropathies with VR1 antagonists. Interfering at the end of the CXCR4/VR1 cascade allows selective inhibition of gp120-mediated neuronal activation and subsequent neuronal cell death protection sparing other essential functions of the upstream signaling cascade.

**Table 4:**

| IC₅₀ ([M]) of indicated inhibitors upon stimulation with gp120 (250pg/ml) using CGRP-release from rat DRG neurons (2.3.2. Method B). | | | | | | | |
|---|---|---|---|---|---|---|---|
| | **PD98059** | **U0126** | **SB203580** | **QN** | **NGDA** | **CAZ** | **Cpd. Example 1** |
| **IC**_{**50**} **[M]** | 9.36E-09 | 1.90E-09. | 2.89E-09 | 1.53E-06 | 4.30E-05 | 1.09E-07 | 4.40E-10 |

### Figure 4: Inhibition of gp120- mediated CGRP-release from rat DRG neurons by PD98059, U0126, SB203580, QN, NDGA, Ibuprofen, Capsazepin and Cpd 1 (2.3.2. Method B).

DRG neurons were isolated from adult male Wistar rats and incubated at 37°C, 5% CO₂ for 36 h at a concentration of 8000 DRG neurons/stimulation point. After washing once with RAB buffer, cells were stimulated for 10 min with gp120 HIV-1_{IIIB} alone (250 pg/ml; black squares) or in the presence of the indicated concentrations of (A) SB203580 (p38 Inh.; white squares), PD98059 (MEK-1 Inh.; black circles) and U0126 (MEK-1 Inh.; white circles); (B) Quinacrine (PLA₂ Inh.; white squares); (C) NDGA (LOX Inh.; white squares); Ibuprofen (COX Inh.; white circles); and (D) Capsazepin (VR1 Inh.; white circles) and Cpd 1 (VR1 Inh.; white squares). Supernatants were removed and analyzed by EIA for CGRP release.

### 4.6 Example 6

CAZ and Cpd 1 are capable to block CGRP release from gp120-stimulated sciatic nerve (Table 5; Figure 5) delineating that VR1 antagonists block gp120-mediated neuronal activation. The data further demonstrate that VR1 antagonists can completely inhibit gp120-mediated CGRP release in peripheral nerves thus propose VR1 as a central target for the interference with HIV-mediated neuropathies and neuropathic pain states.

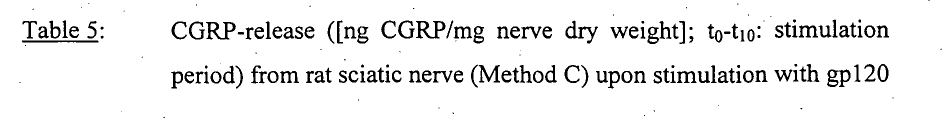

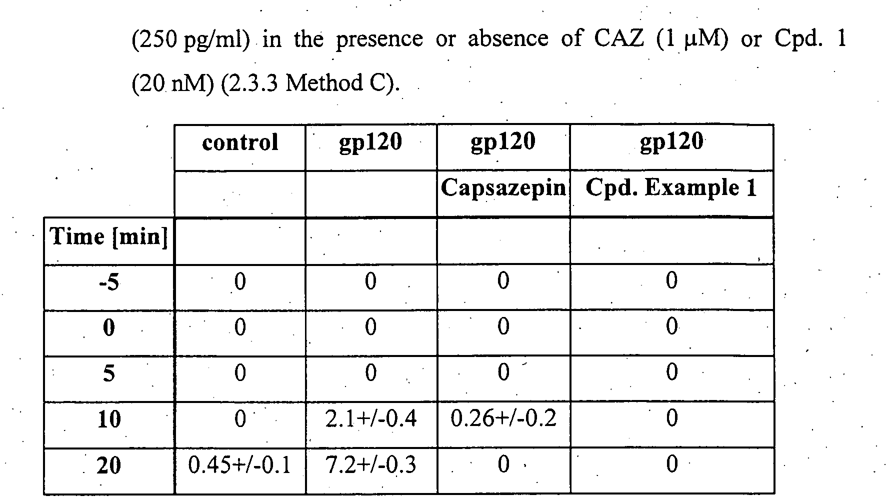

### Figure 5: Inhibition of gp120-mediated CGRP release form rat sciatic nerve by Capsazepin and Cpd 1 (2.3.3. Method C).

Sciatic nerve was isolated from adult male Wistar rats, fixed on a polystyrol rod and maintained in RAB buffer until 5 min before stimulation (time point ― 5). RAB buffer was exchanged, the nerve incubated for 5 min and subsequently stimulated for 10 min with gp120 HIV-1_{IIIB} (250 pg/ml; black squares) or control peptide (250 pg/ml; black crosses) in the presence of Capsazepin (1 µM; white circles), compound of example 1 (20 nM; white squares) or 0.1% DMSO as control (black squares and crosses). After 5 min, 10 min and 20 min supernatant was substituted with fresh solution or RAB buffer pH 7.4 and the samples placed on ice until analysis. All samples were analyzed by EIA for CGRP release and normalized by the dry weight of the particular sciatic nerve.

In summary, data presented here demonstrate for the first time that
(i) HIV envelope protein gp120 induces stimulation of primary DRG neurons and peripheral nerves and the release of the algetic neuropeptide CGRP;
(ii) Neuronal stimulation by gp120 directly modulates the molecular parameters of VR1;
(iii) Gp120-mediated CGRP release can be blocked by MEK-1 inhibitors (PD98059 and U0126), a p38 inhibitor (SB203580), a PLA₂ inhibitor (Quinacrine (QN)), a 12- and 15-LOX inhibitor (Nordihydroguaiaretic Acid (NDGA)) and in particular by VR1 inhibitors (Capsazepin (CAZ) and Cpd 1). In contrast, COX inhibitors (Ibuprofen) can not inhibit gp120-mediated CGRP release from DRG neurons;

Thus, gp120-mediated neuronal stimulation utilizes a novel signaling cascade including, MAPKKs, MAPKs, PLA2, and 12-LOX leading to the production of endovanilloids, such as 12-HPETE and 12-HETE. Upon binding to VR1, endovanilloids induce Ca²⁺ influx from the extracellular space, neuronal depolarization, neuronal activation and the release of neuropeptides, in particular nociceptive neuropeptides such as Substance P [Oh et al. (2001)] and CGRP (Figure 1-5). These neuropeptides are responsible for pain perception *in vivo* [Willis et al. (2001)]. In conclusion, gp120-mediated neuronal stimulation is initiated upon binding of gp120 to CXCR4 but is executed upon activation of VR1.

HIV-associated neuropathic pain, in particular DSP, is comparable to cancer pain and develops in 30% of HIV patients and almost 100% of AIDS patients [Brinley et al. (2001)]. Current therapies are widely unsatisfying due to their low efficacy [Brinley et al. (2001); Glare (2001); Verma (2001); Paice et al. (2000)]. In addition, several drugs frequently used for the treatment of other neuropathic pain states, have been proven ineffective in clinical trails [Paice et al. (2000); Shlay et al. (1998); Kieburtz et al. (1998)]. Thus, the medical need for innovative drugs specifically targeting HIV-mediated nociceptive pathways is exceedingly high. Date presented here, elucidate how HIV stimulates nocicpeptive neurons and provides several potential targets to interfere with HIV-mediated nociception. In particular, VR1 antagonists prove to be highly efficient in blocking HIV-mediated release of nociceptive neuropeptides, thus reducing HIV-associated neuropathic pain. In contrast to Opoids and NSAIDs, which show little efficacy and display several objectionable side effects, VR1 antogonists can be expected highly efficient with few adverse effects [Walker et al. (2003); Rigoni et al. (2003); Pomonis et al. (2003)].

Neuronal cell death frequently occurs upon chronic stimulation leading to pathologically prolonged Ca²⁺ influx [Kaul et al. (2001)]. High intracellular Ca²⁺ concentrations that can not be counterbalanced by transport of Ca²⁺ to the ER, the mitochondria or the extracellular space induce programmed cell death [Jambrina et al. (2003); Leist and Jäättelä (2001)]. It is generally accepted, that pathways leading to neuronal stimulation and neuronal cell death are identical [Kaul et al. (2001); Leist and Jäättelä (2001)]: Dependent on the duration these pathways lead to stimulation or initiation of neuronal cell death [Kaul et al. (2001)]. Neuropathic pain includes chronic neuronal stimulation paired with neurotoxicity [Glare (2001)] and chronic stimulation of VR1 by vanilloids and endovanilloids induces neuronal cell death *in vitro* and *in vivo* [Yamaji et al. (2003); Jambrina E et al. (2003); Herberg U and Sagen J (2001)]. Thus, it is very likely that inhibition, of gp120-induced signaling using VR1 antagonists interferes not only with stimulation of nociceptive neurons but also inhibits neuronal cell death - this means that VR1 antagonists may act as neuroprotective agents in HIV and AIDS patients.

Similar to the PNS, CXCR4 and VR1 are co-expressed in the CNS, particularly in the brain. [Mezey et al. (2000); Kaul et al. (2001)]. Thus, the use of VR1 antagonists may be extended to central HIV-associated neuropathies, such as HIV-associated dementia (HAD) and minor cognitive/motor disorder (MCMD). Highly similar to peripheral gp120-associated neuropathies, HAD and MCMD are characterized by gp120-mediated chronic neuronal stimulation, increased and prolonged intracellular Ca²⁺ concentrations and subsequent neuronal cell death [Kaul et al. (2001)].

In conclusion, the invention relates to the use of VR1 antagonists for the treatment of HIV-associated neuropathies and neuropathic pain states in the PNS and CNS interfering with the nociceptive and neurotoxic properties of HIV.

### 5 REFERENCES

U.S. 4,522,811
Audubert F et al. (1999) Biochim Biophys Acta 1437: 265-276
Barre-Sinoussi F et al. (1983) Science 220 (4599): 868-871
Bhave G et al. (2002) Neuron 15; 35(4): 721-31
Bleul CC et al. (1996) Nature 382: 829-833
Bouhassira D et al. (1999) Pain 80: 265-272
Brannagan 3^{rd} TH et al. (1997) Ann. Neurol. 42: 368-372
Brinley FJ et al. (2001) Arch. Neurol. 58: 1561-1566
Caterina MJ et al. (1997) Nature 3 89: 816-824
Caterina MJ et al. (2000) Science 288: 306-313
Davis JB et al. (2000) Nature 405: 183-187
Deng HK et al. (1996) Nature 381: 661-666
Doranz BJ et al. (1996) Cell 85: 1149-1158
Dragic T et al. (1996) Nature 381: 667-673
Eron JJ et al. (1996) Lancet 348: 1547-1551
Gallo RC et al. (1983) Science 220 (4599): 865-867
Gelmann EP et al. (1983) Science 220 (4599): 862-865
Glare PA (2001) Europ. J. Pain 5 (Suppl. A): 43-48
Griffin JW et al. (1998) The neurology of AIDS; pp 275-291, Chapman&Hall, New York
Hesselgesser J et al. (1997) Curr. Biol. 7: 112-121
Herberg U and Sagen J (2001) J Neuroimmunol. 116: 29-39
Hiura A et al. (2002) Anat Sci Int. 77 (1): 47-50
Hewitt DJ et al (1997) Pain 70: 117-123
Hui K et al. (2003) Biophys J. 84(5): 2957-68
Hwang SW et al. (2000) PNAS 97:6155-6160
Jambrina E et al. (2003) J Biol Chem. 278 (16): 14134-45
Janeway & Travers (1997) Immunobiology; 3^{rd} edition, Current Biology Ltd, New York
Kaul M et al. (2001) Nature 410: 988-998
Kedei N et al. (2001) J Biol Chem. 276 (30): 28613-9
Kieburtz K et al. (1998) Neurology 51 (6): 1682-8
Kim CS et al. (2003) Cell Signal. 15 (3): 299-306
Kelly S et al. (2002) Brain Res. 935 (1-2):103-8
Kuzhikandathil EV (2001) J Neurosci. 21 (22): 8697-706
Leist M and Jäättelä M (2001) Nat. Mol. Cell Biol. Rev. 2: 1-10
Mezey E et al. (2000) Proc Natl Acad Sci U S A. 97(7): 3655-60
Miller AL et al. (1994) Methods Cell Biol. 40: 305-38
Miller RJ and Meucci O (1999) Trends. Neurosci. 22: 471-479
Milligan ED et al. (2000) Brain Res. 861: 105-116
Oberlin E et al. (1996) Nature 382: 833-835
Oh SB et al. (2001) J. Neurosci. 21 (14): 5027-5035
O'Malley DM et al. (1999) Methods Mol Biol. 122:261-303.
Paice JA et al. (2000) J. Pain Symptom. Manage. 19 (1): 45-52
Pardo CA et al. (2001) J. PNS 6: 21-27
Pomonis JD et al. (2003) J Pharmacol Exp in press
Rigoni Metal. (2003) Br J Pharmacol. 138(5): 977-85
Sancho R et al. (2002) Eur J Immunol. 32 (6): 1753-63
Sauer SK et al. (2001) Eur J Neurosci 14 (8):1203-1208
Shin J et al. (2002) PNAS 23: 10150-10155
Shlay JC et al. (1998) JAMA.280 (18): 1590-5
Stantchev TS and Broder CC (2001) Cytokine & Growth Factor Reviews 12: 219-243
Tominaga M et al. (1998) Neuron 21: 531-543
Verma A (2001) J. PNS 6: 8-13
Walker KM (2003) J Pharmacol Exp Ther. 304(1): 56-62
Willis AD (2001) Ann N Y Acad Sci. 933: 142-56
Yamaji K et al. (2003) Thromb Haemost. 89(5): 875-84
Zhu M et al. (1999) J. Neurosci. 18: 679-686

### 6 ABREVIATIONS

- AA: arachidonic acid
- AC: Adenylyl Cyclase
- AIDS: Acquired Immune Deficiency Syndrome
- BSA: Bovine serum albumin
- C: Cysteine
- CAPS: Capsaicin
- CAZ: Capsazepin
- CCR: CC Chemokine receptor
- CD: Cluster of differentiation
- CGRP: calcitonin gene-related peptide
- CMV: Cytomegalovirus
- CNS: Central nervous system
- COX: Cyclooxygenase
- CXCR: CXC chemokines receptor
- DAG: 1,2-*sn*-Diacylglycerol
- DMSO: Dimethylsulfoxide
- DNA: Desoxyribonucleic acid
- DRG: Dorsal root ganglion
- DSP: distal sensory painful polyneuropathy
- EC: Efficiency concentration
- EIA: Enzyme immuno assay
- ERK: Extracellular-signal regulated kinase
- et al.: *et alii*
- FCA: Freund's complete adjuvant
- FIA: Freund's incomplete adjuvans
- Gp: Glycoprotein
- GPCR: G-protein-coupled receptor
- HAD: HIV-associated dementia
- HHV: Human Herpes Virus
- HIV: Human Immunodeficiency Virus
- HPETE: hydroperoxyeicosatetraenoic acid
- hu: human
- HZV: Herpes zoster virus
- I: Inositol
- IBU: Ibuprofen
- IC: Inhibitory concentration
- Ig: Immunoglobulin
- LOX: Lipoxygenase
- LT: Leukotriene
- MAPK: mitogen-activated protein kinase
- MCMD: minor cognitive/motor disorder
- MEK: mitogen-activated protein kinase kinase
- MIP: macrophage inhibitory protein
- mu: murine
- MW: Molecular weight
- N₂: Nitrogen
- N₂O: Dinitrogenoxide
- NDGA: Nordihydroguaiaretic acid
- O₂: Oxygen
- ON: over night
- QN: Quinarine
- PBS: Phosphate buffered saline
- pH: *potentia hydrogeni*
- PI: Phosphatidylinositol
- PKA: Protein Kinase A
- PKB: Protein Kinase B
- PKC: Protein Kinase C
- PLA: Phospholipase A
- PLC: Phospholipase C
- RAB: Release assay buffer
- RT: Room temperature (20-25°C)
- SDF: Stroma-derived factor
- SN: supernatant
- VR: Vanilloid receptor
- v/v: Volume per volume
- v/w: Volume per weight

## Claims

1. Methods of screening for VR1 antagonists useful for the treatment of HIV-mediated neuropathy or HIV-mediated pain.

2. Methods of screening for VR1 antagonists useful for the treatment of HIV-mediated neuropathy or HIV-mediated pain using Ca²⁺-sensitive dyes of bioluminescence assays in cell-free or cellular assay systems.

3. The use of a VR1 antagonist for the preparation of a pharmaceutical composition for the treatment of HIV-mediated neuropathy or HIV-mediated pain.

4. The use according to claim 3, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

5. A pharmaceutical composition containing a VR1 antagonist for the treatment of HIV-mediated neuropathy or HIV-mediated pain.

6. The pharmaceutical composition according to claim 5, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

7. A pharmaceutical composition containing a VR1 antagonist for the treatment of HIV-mediated neuropathy or HIV-mediated pain where the active ingredient is the compound 1:

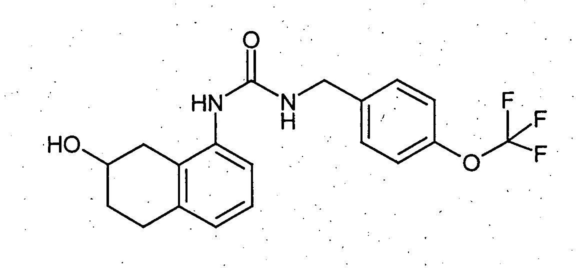

8. The pharmaceutical composition according to claim 7, wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

9. The use of a VR1 antagonist for the regulation of VR1 activity in a subject having HIV-mediated neuropathy or HIV-mediated pain.

10. The use according to claim 9, wherein the VR1 antagonist is compound 1:

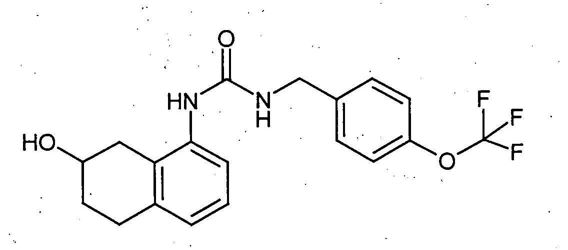

11. The use of a 12-LOX antagonist for the preparation of a pharmaceutical composition for the treatment of HIV-mediated neuropathy or HIV-mediated pain.

12. The use according to claim 11 wherein HIV-mediated neuropathy or pain is caused by a disorder comprised in a group of disorders consisting of acute and chronic inflammatory demyelinating polyneuropathy, diffuse infiltrative lymphocytosis syndrome 'with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

13. The use of a PLA₂ antagonist for the preparation of a pharmaceutical composition for the treatment of HIV-mediated neuropathy or HIV-mediated pain.

14. The use according to claim 13 wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

15. The use of a MAPK antagonist for the preparation of a pharmaceutical composition for the treatment of HIV-mediated neuropathy or HIV-mediated pain.

16. The use according to claim 15 wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

17. The use of a CXCR4 antagonist for the preparation of a pharmaceutical composition for the treatment of HIV-mediated neuropathy or HIV-mediated pain.

18. The use according to claim 17 wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.

19. The use of an antagonist or modulator interfering upstream of CXCR4 ligands for the preparation of a pharmaceutical composition for the treatment of HIV-mediated neuropathy or HIV-mediated pain.

20. The use according to claim 19 wherein HIV-mediated neuropathy or pain is caused by a disorder comprised in a group of disorders consisting of acute and chronic inflammatory demyelinating polyneuropathy, diffuse infiltrative lymphocytosis syndrome with neuropathy, mononeuropathy multiplex, progressive polyneurophathy, Cytomegalovirus (CMV)-related neuropathies, Herpes zoster virus (HZV)-related neuropathies, distal sensory painful polyneuropathy (DSP), and HIV-associated dementia.
